# EUROPÄISCHE PATENTSCHRIFT

(11) **EP 0 583 499 B1**
(45) Veröffentlichungstag und Bekanntmachung des Hinweises auf die Patenterteilung: **06.03.1996**
(21) Anmeldenummer: 92114090.1
(22) Anmeldetag: 18.08.1992
(51) Int. Cl.: A61B 5/05, A61N 1/39, A61N 1/365

(54) **Verfahren zum Detektieren von Herzkammerflimmern und Vorrichtung zum Detektieren und Behandeln von Herzkammerflimmern**
Method for detecting cardial ventricular fibrillation and means for detection and treating of cardial ventricular fibrillation
Procédé pour détecter la fibrillation cardiaque ventriculaire et dispositif pour détecter et traitement de fibrillation cardiaque ventriculaire

(43) Veröffentlichungstag der Anmeldung: 23.02.1994
(73) Patentinhaber: Pacesetter AB, S-171 95 Solna (SE)
(72) Erfinder: Hagel, Pia, S-191 50 Sollentuna (SE); Högnelid, Kurt, S-137 38 Västerhaninge (SE); Norén, Kjell, S-171 58 Solna (SE)
(74) Vertreter: Lettström, Richard Wilhelm

(56) Entgegenhaltungen:
- EP-A- 0 074 126
- GB-A- 2 070 282
- US-A- 5 058 583

## Beschreibung

Die Erfindung betrifft ein Verfahren zum Detektieren von Herzkammerflimmern durch Auswertung eines vom Blutvolumen im Herzen abhängigen Impedanzmeßsignals. Die Erfindung betrifft ferner eine Vorrichtung zum Detektieren und Behandeln von Herzkammerflimmern mit einer Impedanzmeßeinrichtung zum Erfassen eines vom Blutvolumen im Herzen abhängigen Impedanzmeßsignals, einer Auswerteeinrichtung zur Auswertung des Impedanzmeßsignals und einer von der Auswerteeinrichtung zur Erzeugung von elektrischen Impulsen aktivierbaren impulserzeugenden Einrichtung, an der ausgangsseitig eine Elektrodenanordnung zur Überführung der elektrischen Impulse an das Herz angeschlossen ist.

Herzkammerflimmern ist eine von mehreren Rhythmusstörungen des Herzens mit unnatürlich überhöhter Frequenz verursacht durch Veränderungen der Erregungsbildung oder Erregungsleitung im Herzen. Derartige Rhythmusstörungen oder sogenannte Tachyarrhythmien führen zu einer reduzierten Kammerfüllung und Auswurfleistung des Herzens und können im schlimmsten Fall, nämlich bei Vorliegen von Herzkammerflimmern zu einem Stillstand der Blutförderung durch das Herz führen. Um zur Behandlung der unterschiedlichen Arten von Tachyarrhythmien geeignete Therapieverfahren wie antitachykarde Stimulation, Kardioversion oder Defibrillierung anwenden zu können, ist zunächst eine Erkennung der jeweiligen Rhythmusstörung des Herzens erforderlich. Dabei kann die Detektion von Tachyarrhythmien ausschließlich mit Hilfe des intrakardiellen Elektrogramms und nachfolgender Auswertung herzschlagfrequenzbezogener Kriterien wie Frequenz, plötzlicher Frequenzanstieg und anhaltend hoher Frequenzwert problematisch sein.

Aus der EP-A-0 009 255 ist es bekannt, Herzkammerflimmern durch Auswertung sowohl der elektrischen als auch der mechanischen Aktivität des Herzens zu detektieren und im Anschluß an die Detektion des Herzkammerflimmerns eine Defibrillation des Herzens auszulösen. Zur Erfassung der elektrischen Aktivität des Herzens wird von diesem mittels einer Elektrodenanordnung das intrakardielle Elektrogramm abgeleitet. Die mechanische Aktivität des Herzens wird in der Weise ermittelt, daß mittels der Elektrodenanordnung und einer daran angeschlossenen Impedanzmeßeinrichtung ein vom Blutvolumen im Herzen abhängiges Impedanzmeßsignal erfaßt wird und daß die in Abhängigkeit von den Kontraktionen des Herzens erfolgenden Änderungen des Impedanzmeßsignals ausgewertet werden.

Um die Änderungen des Impedanzmeßsignals in Abhängigkeit von den Kontraktionen des Herzens ermitteln zu können, muß eine bezogen auf die Herzfrequenz hohe Anzahl von einzelnen Meßwerten erfaßt und ausgewertet werden.

Der Erfindung liegt die Aufgabe zugrunde, mit einem minimalen Meßaufwand eine sichere Detektion von Herzkammerflimmern zu ermöglichen.

Gemäß der Erfindung wird die Aufgabe dadurch gelöst, daß bei den Verfahren der eingangs angegebenen Art ein Absinken des Niveaus des Impedanzmeßsignals unter einen vorgegebenen Schwellenwert zur Detektion des Herzkammerflimmerns herangezogen wird.

Bezüglich der eingangs genannten Vorrichtung wird die Aufgabe dadurch gelöst, daß die Auswerteeinrichtung eine Detektoreinrichtung zum Detektieren eines Absinkens des Niveaus des Impedanzmeßsignals unter einen vorgegebenen Schwellenwert aufweist.

Bei der Erfindung wird in vorteilhafter Weise der Effekt ausgenutzt, daß sich das Herz beim Vorliegen von Herzkammerflimmern mit Blut füllt, so daß das allgemeine Impedanzniveau absinkt. Mit der Erfassung des Niveaus des Impedanzmeßsignal gelingt es also, Herzkammerflimmern sicher zu detektieren und von anderen Tachyarrhythmien zu unterscheiden. Darüberhinaus ist das Niveau des Impedanzmeßsignals meßtechnisch besonders einfach zu erfassen, weil im Vergleich zur Erfassung der Änderungen des Impedanzmeßsignals nur sehr wenige Meßwerte benötigt werden.

Um den Einfluß von längerfristigen Schwankungen des Niveaus des Impedanzmeßsignals auf die Detektion von Herzkammerflimmern auszuschließen, ist in vorteilhafter Weise vorgesehen, daß ein zeitlicher Mittelwert des Niveaus des Impedanzmeßsignals ermittelt wird und daß der Schwellenwert auf einen um einen vorgegebenen Betrag unter dem Mittelwert liegenden Wert eingestellt wird. In entsprechender Weise ist bei der erfindungsgemäßen Vorrichtung vorgesehen, daß die Auswerteeinrichtung einen Mittelwertbildner zur laufenden Bildung eines zeitlichen Mittelwerts des Niveaus des Impedanzmeßsignals und zur Einstellung des Schwellenwerts auf einen um einen vorgegebenen Betrag unter dem Mittelwert liegenden Wert aufweist.

Die Sicherheit, mit der das Vorliegen von Herzkammerflimmern detektiert wird, läßt sich in vorteilhafter Weise dadurch erhöhen, daß die Geschwindigkeit, mit der das Niveau des Impedanzmeßsignals absinkt, als weiteres Kriterium zur Detektion des Herzkammerflimmerns herangezogen wird. Eine entsprechende Weiterbildung der erfindungsgemäßen Vorrichtung besteht darin, daß die Auswerteeinrichtung eine weitere Detektoreinrichtung zum Detektieren der Geschwindigkeit, mit der das Niveau des Impedanzmeßsignals absinkt, aufweist und daß Mittel zum logischen Verknüpfen der von der Detektoreinrichtung und der weiteren Detektoreinrichtung erzeugten Ausgangssignale in der Weise vorgesehen sind, daß das gleichzeitige Vorliegen beider Ausgangssignale das Herzkammerflimmern anzeigt. Dabei können die Mittel zur logischen Verknüpfung der Ausgangssignale aus einem UND-Glied bestehen, oder es kann vorgesehen sein, daß eine der beiden Detektoreinrichtungen durch das Ausgangssignal der anderen Detektoreinrichtung aktiviert wird.

Zur weiteren Erhöhung der Detektionssicherheit wird in vorteilhafter Weise die Herzfrequenz als zusätzliches Kriterium zur Detektion des Herzkammerflimmerns erfaßt und ausgewertet. Bei der erfindungsgemäßen Vorrichtung ist hierzu ein Herzschlagdetektor vorgesehen, dem eine weitere Auswerteeinrichtung zur Bestimmung der Herzfrequenz nachgeordnet ist, wobei die Ausgangssignale der beiden Auswerteeinrichtungen derart logisch miteinander verknüpft sind, daß das gleichzeitige Vorliegen beider Ausgangssignale das Herzkammerflimmern anzeigt.

Die meßtechnische Erfassung des Niveaus des Impedanzmeßsignals erfolgt auf besonders einfache Weise dadurch, daß die Impedanzmeßeinrichtung Mittel zum Ermitteln des Impedanzmeßsignals aus von der impulserzeugenden Einheit über die Elektrodenanordnung an das Herz abgegebenen elektrischen Impulsen aufweist. Wenn dabei eine Stimulierung des Herzens ausgeschlossen werden soll, weisen die zur Ermittlung des Impedanzmeßsignals erzeugten elektrischen Impulse vorzugsweise eine unter der Reizschwelle des Herzens liegende Impulshöhe auf. Im einfachsten Fall besteht dabei die impulserzeugende Einheit aus einem Defibrillierungsimpulsgenerator, der zur Ermittlung des Impedanzmeßsignals Meßimpulse mit geringstmöglicher Energie erzeugt und nach einer Detektion von Herzkammerflimmern zur Abgabe von energiereichen Defibrillierungsimpulsen aktiviert wird.

Bei einer alternativen, besonders vorteilhaften Weiterbildung der erfindungsgemäßen Vorrichtung weist die impulserzeugende Einheit einen Stimulationsimpulsgenerator zur Abgabe von Herzschrittmacherimpulsen und einen durch die Auswerteeinrichtung aktivierbaren Defibrillierungsimpulsgenerator zur Abgabe von Defibrillierungsimpulsen auf, wobei die von dem Stimulationsimpulsgenerator erzeugten elektrischen Impulse den Mitteln zum Ermitteln des Impedanzmeßsignals aus den elektrischen Impulsen zugeführt sind. Es ist zwar aus der US-A-4 697 591 bereits bekannt, Herzschrittmacherimpulse zur Erlangung eines atmungsabhängigen Impedanzmeßsignals auszuwerten, jedoch ist es durch den Gegenstand der Erfindung erstmals möglich, dieses Meßprinzip und die mit ihm verbundenden Vorteile für die Detektion von Herzkammerflimmern über den Weg der Impedanzmessung anzuwenden. Dagegen ist es bei dem aus der eingangs bereits genannten EP-A-0 009 255 bekannten Verfahren nicht möglich, Herzschrittmacherimpulse zur Bestimmung des Impedanzmeßsignals heranzuziehen, weil für die Auswertung der Änderungen des Impedanzmeßsignals in Abhängigkeit von der Herzpumptätigkeit erheblich mehr Meßwerte erforderlich sind.

Zur Erläuterung der Erfindung wird im folgenden auf die Figuren der Zeichnung Bezug genommen; im einzelnen zeigen
- FIG. 1: ein Ausführungsbeispiel für die erfindungsgemäße Vorrichtung zur Detektion und Behandlung von Herzkammerflimmern und
- FIG. 2: ein Beispiel für den Verlauf eines vom Blutvolumen im Herzen abhängigen Impedanzmeßsignals bei normaler Herztätigkeit und bei Herzkammerflimmern.

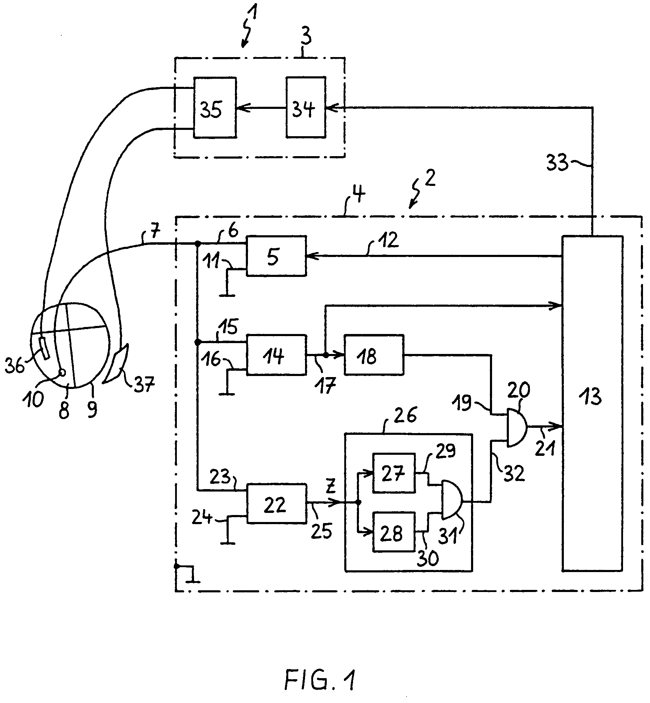

Figur 1 zeigt das Blockschaltbild eines kombinierten Defibrillators 1 und Herzschrittmachers 2, die beide in einem gemeinsamen implantierbaren Gehäuse angeordnet sein können oder, wie hier gezeigt, einzeln für sich jeweils in einem implantierbaren Gehäuse 3 bzw. 4 angeordnet sind.

Der Herzschrittmacher 2 enthält einen Stimulationsimpulsgenerator 5, der an einem Ausgangsanschluß 6 über eine Elektrodenleitung 7 mit einer im Ventrikel 8 des Herzens 9 angeordneten Stimulationselektrode 10 verbunden ist. Der zweite Ausgangsanschluß 11 des Stimulationsimpulsgenerators 5 ist mit dem Gehäuse 4 des Herzschrittmachers 2 verbunden, das als Gegenelektrode zur Stimulationselektrode 10 dient. Der Stimulationsimpulsgenerator 5 ist über eine Steuerleitung 12 mit einer Herzschrittmachersteuerung 13 verbunden, die über die Steuerleitung 12 die Abgabe von Stimulationsimpulsen durch den Stimulationsimpulsgenerator 5 veranlaßt. Ein Herzschlagdetektor 14 ist zur Detektion von stimulierten oder natürlichen elektrischen Herzaktivitäten mit einem ersten Eingangsanschluß 15 an der Stimulationselektrode 10 und mit einem zweiten Eingangsanschluß 16 an dem Gehäuse 4 des Herzschrittmachers 2 angeschlossen. Zur Steuerung der Funktion des Herzschrittmachers 2 in Abhängigkeit von den detektierten elektrischen Herzaktivitäten ist der Herzschlagdetektor 14 an seinem Ausgang 17 mit der Herzschrittmachersteuerung 13 verbunden. An dem Ausgang 17 des Herzschlagdetektors 14 ist ferner eine Auswerteeinrichtung 18 angeschlossen, die die detektierten elektrischen Herzaktivitäten bezüglich ihrer Frequenz auswertet. Die Auswerteeinrichtung 18 ist ausgangsseitig mit einem ersten Eingang 19 eines UND-Glieds 20 verbunden, das mit seinem Ausgang 21 an der Herzschrittmachersteuerung 13 angeschlossen ist.

Eine Spannungsmeßeinrichtung 22 ist mit einem ersten Eingangsanschluß 23 an der Stimulationselektrode 10 und mit einem zweiten Eingangsanschluß 24 an dem Gehäuse 4 des Herzschrittmachers 2 angeschlossen. Die Spannungsmeßeinrichtung 22 bildet zusammen mit dem Stimulationsimpulsgenerator 5 eine Impedanzmeßeinrichtung, in der der Abfall der Impulshöhe der von dem Stimulationsimpulsgenerator 5 erzeugten Stimulationsimpulse zwischen dem Anfang eines jeden Stimulationsimpulses und dem Ende des betreffenden Stimulationsimpulses ermittelt wird und daraus ein transkardiales Impedanzmeßsignal Z am Ausgang 25 der Spannungsmeßeinrichtung 22 abgeleitet wird. An dem Ausgang 25 der Spannungsmeßeinrichtung 22 ist eine Auswerteeinrichtung 26 angeschlossen, die eine erste Detektoreinrichtung 27 zum Detektieren eines Absinkens des Niveaus des Impedanzmeßsignals Z unter einen vorgegebenen Schwellenwert enthält und eine zweite Detektoreinrichtung 28 aufweist, in der die Geschwindigkeit, mit der sich das Niveau des Impedanzmeßsignals Z ändert auf Überschreiten einer Mindestgeschwindigkeit überwacht wird. Die beiden Detektoreinrichtungen 27 und 28 sind an ihren Ausgängen 29 und 30 über ein weiteres UND-Glied 31 mit einem zweiten Eingang 32 des UND-Glieds 20 verbunden.

Der Herzschrittmachersteuerung 13 wird also über den Ausgang 21 des UND-Glieds 20 dann das Vorliegen von Herzkammerflimmern mitgeteilt, wenn die Herzfrequenz einen vorgegebenen Wert überschreitet und das Niveau der transkardialen Impedanz mit einer Mindestgeschwindigkeit, also beispielsweise innerhalb eines vorgegebenen Zeitfensters, eine vorgegebenen Schwellenwert unterschreitet.

Die Herzschrittmachersteuerung 13 gibt daraufhin über eine Steuerleitung 33 oder eine hierzu alternative drahtlose Signalübertragungsstrecke ein Steuersignal an eine Steuereinrichtung 34 des Defibrillators 1 ab. Die Steuereinrichtung 34 aktiviert daraufhin einen Defibrillierungsimpulsgenerator 35 zur Abgabe eines Defibrillierungsimpulses über zwei Defibrillierungselektroden 36 und 37 an das Herz 9.

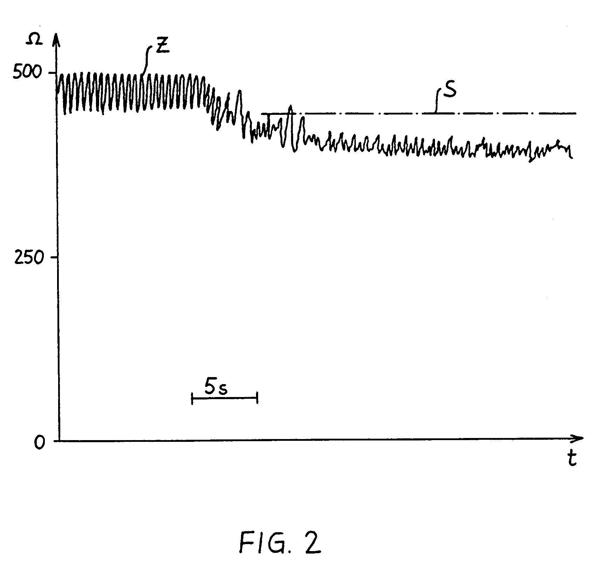

Figur 2 zeigt ein Beispiel für den Verlauf des zwischen der Stimulationselektrode 10 und dem Gehäuse 4 des Herzschrittmachers 2 erfaßten Impedanzmeßsignals Z bei natürlicher Herzaktivität und sich daran anschließendem Herzkammerflimmern. Wie die Figur zeigt, ändert sich das Impedanzmeßsignal Z während der natürlichen Herzaktivität in Abhängigkeit von den Kontraktionen des Herzmuskels. Beim Auftreten von Herzkammerflimmern verändert sich die Pumpaktivität des Herzens zu einem hochfrequenten, unkoordinierten Zucken des Myocards, weswegen die Frequenz der Änderungen des Impedanzmeßsignals Z zunimmt und die Amplituden der Änderungen des Impedanzsignals Z abnehmen. Diese Erscheinungen werden bei dem aus der eingangs genannten EP-A-0 009 255 zur Auswertung der mechanischen Aktivität des Herzens herangezogen. Figur 2 zeigt aber auch, daß das allgemeine Niveau des Impedanzmeßsignals Z beim Auftreten von Herzkammerflimmern plötzlich absinkt. Dies ist darauf zurückzuführen, daß der Herzmuskel bei Herzkammerflimmern das Blut nicht mehr pumpt, sondern statt dessen sich mit Blut füllt. Bei der in Figur 1 gezeigten Vorrichtung wird dieser Effekt ausgenutzt, indem das plötzliche Absinken des Niveaus des Impedanzmeßsignals Z unter den vorgegebenen Schwellenwert S als ein Kriterium zur Detektion des Herzkammerflimmerns herangezogen wird. Um das Niveau des Impedanzmeßsignals Z zu bestimmen ist es völlig ausreichend, wenn mit einer geringen, z.B. der Stimulationsfrequenz des Stimulationsimpulsgenerators 5 in Figur 1 entsprechenden Abtastfrequenz Meßwerte der transkardialen Impedanz erfaßt werden und ein laufender Mittelwert über einige wenige, z.B. fünf der erfaßten Meßwerte gebildet wird, um den Einfluß der rauschartigen Änderungen des Impedanzmeßsignals Z auf die Detektion eines Unterschreitens des Schwellenwerts S auszuschalten. Anstelle dieser Kurzzeitmittelwertbildung kann auch vorgesehen werden, daß eine bestimmte Anzahl von unmittelbar aufeinander folgenden Meßwerten den Schwellenwert S unterschritten haben muß, um daraus ein Absinken des Niveaus des Impedanzsignals Z unter den Schwellenwert S zu detektieren.

Um den Schwellenwert S an längerfristige Schwankungen oder Änderungen des Impedanzniveaus anpassen zu können, wird in der Auserteeinrichtung 26 zusätzlich ein langfristiger Mittelwert aus den Meßwerten des Impedanzmeßsignals Z gebildet und der Schwellenwert S auf einen den Mittelwert um einen vorgegebenen Betrag unterschreitenden Wert eingestellt.

Alternativ zu dem in Figur 1 gezeigten Ausführungsbeispiel der erfindungsgemäßen Vorrichtung können anstelle der von dem Stimulationsimpulsgenerator 5 abgegebenen Herzschrittmacherimpulsen auch von dem Defibrillationsimpulsgenerator 35 erzeugte energiearme elektrische Impulse zur Messung der transkardialen Impedanz herangezogen werden, wobei dann die Spannungsmeßeinrichtung 22 an einer oder beiden Defibrillationselektroden 36 und 37 angeschlossen ist. Schließlich kann auch eine von den impulserzeugenden Einrichtungen 5 und 35 unabhängige Impedanzmeßvorrichtung vorgesehen sein.

### Bezugszeichenliste

- 1: Defibrillator
- 2: Herzschrittmacher
- 3: Gehäuse von 1
- 4: Gehäuse von 2
- 5: Stimulationsimpulsgenerator
- 6: erster Ausgangsanschluß von 5
- 7: Elektrodenleitung
- 8: Ventrikel
- 9: Herz
- 10: Stimulationselektrode
- 11: zweiter Ausgangsanschluß von 5
- 12: Steuerleitung
- 13: Herzschrittmachersteuerung
- 14: Herzschlagdetektor
- 15,16: Eingangsanschlüsse von 14
- 17: Ausgang von 14
- 18: Auswerteeinrichtung
- 19: erster Eingang von 20
- 20: UND-Glied
- 21: Ausgang von 20
- 22: Spannungsmeßeinrichtung
- 23,24: Eingangsanschlüsse von 22
- 25: Ausgang von 22
- 26: Auswerteeinrichtung
- 27,28: Detektionseinrichtung
- 29,30: Ausgänge von 27,28
- 31: weiteres UND-Glied
- 32: zweiter Eingang von 20
- 33: Steuerleitung
- 34: Steuereinrichtung
- 35: Defibrillierungspulsgenerator
- 36,37: Defibrillierungselektrode
- S: Schwellenwert
- Z: Impedanzmeßsignal

## Patentansprüche

1. Verfahren zum Detektieren von Herzkammerflimmern durch Auswertung eines vom Blutvolumen im Herzen (9) abhängigen Impedanzmeßsignals (Z), **dadurch gekennzeichnet,** daß ein Absinken des Niveaus des Impedanzmeßsignals (Z) unter einen vorgegebenen Schwellenwert (S) zur Detektion des Herzkammerflimmerns herangezogen wird.

2. Verfahren nach Anspruch 1, **dadurch gekennzeichnet,** daß ein zeitlicher Mittelwert des Niveaus des Impedanzmeßsignals (Z) ermittelt wird und daß der Schwellenwert (S) auf einen um einen vorgegebenen Betrag unter dem Mittelwert liegenden Wert eingestellt wird.

3. Verfahren nach Anspruch 1, **dadurch gekennzeichnet,** daß die Geschwindigkeit, mit der das Niveau des Impedanzmeßsignals (Z) absinkt, als ein weiteres Kriterium zur Detektion des Herzkammerflimmerns herangezogen wird.

4. Verfahren nach Anspruch 1 oder 2, **dadurch gekennzeichnet,** daß als zusätzliches Kriterium zur Detektion des Herzkammerflimmerns die Herzfrequenz erfaßt und ausgewertet wird.

5. Vorrichtung zum Detektieren und Behandeln von Herzkammerflimmern mit einer Impedanzmeßeinrichtung (5,22) zum Erfassen eines vom Blutvolumen im Herzen (9) abhängigen Impedanzmeßsignals (Z), einer Auswerteeinrichtung (26) zur Auswertung des Impedanzmeßsignals (Z) und einer von der Auswerteeinrichtung (26) zur Erzeugung von elektrischen Impulsen aktivierbaren impulserzeugenden Einrichtung (1), an der ausgangsseitig eine Elektrodenanordnung (36,37) zur Überführung der elektrischen Impulse an das Herz (9) angeschlossen ist, **dadurch gekennzeichnet,** daß die Auswerteeinrichtung (26) eine Detektoreinrichtung (27) zum Detektieren eines Absinkens des Niveaus des Impedanzmeßsignals (Z) unter einen vorgegebenen Schwellenwert (S) aufweist.

6. Vorrichtung nach Anspruch 5, **dadurch gekennzeichnet,** daß die Auswerteeinrichtung (26) einen Mittelwertbildner zur laufenden Bildung eines zeitlichen Mittelwerts des Niveaus des Impedanzmeßsignals (Z) und zur Einstellung des Schwellenwerts (S) auf einen um einen vorgegebenen Betrag unter dem Mittelwert liegenden Wert aufweist.

7. Vorrichtung nach Anspruch 5 oder 6, **dadurch gekennzeichnet,** daß die Auswerteeinrichtung (26) eine weitere Detektoreinrichtung (28) zum Detektieren der Geschwindigkeit, mit der das Niveau des Impedanzmeßsignals (Z) absinkt, aufweist und daß Mittel (31) zur logischen Verknüpfung der von der Detektoreinrichtung (27) und der weiteren Detektoreinrichtung (28) erzeugten Ausgangssignale in der Weise vorgesehen sind, daß das gleichzeitige Vorliegen beider Ausgangssignale das Herzkammerflimmern anzeigt.

8. Vorrichtung nach einem der Ansprüche 5 bis 7, **dadurch gekennzeichnet,** daß ein Herzschlagdetektor (14) vorgesehen ist, dem eine weitere Auswerteeinrichtung (18) zur Bestimmung der Herzfrequenz nachgeordnet ist, und daß die Ausgangsignale der beiden Auswerteeinrichtungen (18,26) derart logisch miteinander verknüpft sind, daß das gleichzeitige Vorliegen beider Ausgangssignale das Herzkammerflimmern anzeigt.

9. Vorrichtung nach einem der Ansprüche 5 bis 8, **dadurch gekennzeichnet,** daß die Impedanzmeßeinrichtung (5,22) Mittel (22) zum Ermitteln des Impedanzmeßsignals (Z) aus von der impulserzeugenden Einheit (5) über die Elektrodenanordnung (10,4) an das Herz (8) abgegebenen elektrischen Impulsen aufweist.

10. Vorrichtung nach Anspruch 9, **dadurch gekennzeichnet,** daß die zur Ermittlung des Impedanzmeßsignals (Z) erzeugten elektrischen Impulse eine unter der Reizschwelle des Herzens (9) liegende Impulshöhe aufweisen.

11. Vorrichtung nach Anspruch 9 oder 10, **dadurch gekennzeichnet,** daß die impulserzeugende Einheit einen Stimulationsimpulsgenerator (5) zur Abgabe von Herzschrittmacherimpulsen und einen durch die Auswerteeinrichtung (26) aktivierbaren Defibrillierungsimpulsgenerator (35) zur Abgabe von Defibrillierungsimpulsen aufweist und daß die von dem Stimulationsimpulsgenerator (5) erzeugten elektrischen Impulse den Mitteln (22) zum Ermitteln des Impedanzmeßsignals (Z) aus den elektrischen Impulsen zugeführt sind.

## Claims

1. Method for detecting ventricular flickering by evaluating an impedance measuring signal (Z) dependent on the blood volume in the heart (9), characterized in that a lowering of the level of the impedance measuring signal (Z) below a specified threshold value (S) is used in order to detect the ventricular flickering.

2. Method according to claim 1, characterized in that a temporal mean value of the level of the impedance measuring signal (Z) is determined and in that the threshold value (S) is adjusted to a value lying below the mean value by a specified amount.

3. Method according to claim 1, characterized in that the speed at which the level of the impedance measuring signal (Z) drops is used as an additional criterion to detect the ventricular flickering.

4. Method according to claim 1 or 2, characterized in that the heart rate is detected and evaluated as an additional criterion to detect the ventricular flickering.

5. Device for detecting and treating ventricular flickering with an impedance measuring device (5, 22) to record an impedance measuring signal (Z) dependent on the blood volume in the heart (9), an evaluation device (26) to evaluate the impedance measuring signal (Z) and a pulse-generating device (1) which can be activated by the evaluation device (26) to generate electrical pulses, to which device (1) there is connected on the output side an electrode arrangement (36, 37) to convey the electrical pulses to the heart (9), characterized in that the evaluation device (26) has a detector device (27) to detect a lowering of the level of the impedance measuring signal (Z) below a specified threshold value (S).

6. Device according to claim 5, characterized in that the evaluation device (26) has a mean-value former for the continuous formation of a temporal mean value of the level of the impedance measuring signal (Z) and for the adjustment of the threshold value (S) to a value lying below the mean value by a specified amount.

7. Device according to claim 5 or 6, characterized in that the evaluation device (26) has an additional detector device (28) to detect the speed at which the level of the impedance measuring signal (Z) drops, and in that means (31) are provided for the logical connection of the output signals, which are generated by the detector device (27) and the additional detector device (28), in such a way that the simultaneous presence of both output signals indicates the ventricular flickering.

8. Device according to one of claims 5 to 7, characterized in that a heart-beat detector (14) is provided, after which there is arranged an additional evaluation device (18) to determine the heart rate, and in that the output signals of the two evaluation devices (18, 26) are connected logically to each other in such a way that the simultaneous presence of both output signals indicates the ventricular flickering.

9. Device according to one of claims 5 to 8, characterized in that the impedance measuring device (5, 22) has means (22) to determine the impedance measuring signal (Z) from electrical pulses emitted by the pulse-generating unit (5) by way of the electrode arrangement (10, 4) to the heart (8).

10. Device according to claim 9, characterized in that the electrical pulses generated to determine the impedance measuring signal (Z) have a pulse level lying below the stimulus threshold of the heart (9).

11. Device according to claim 9 or 10, characterized in that the pulse-generating unit has a stimulation pulse generator (5) for the emission of heart pacemaker pulses and has a defibrillation pulse generator (35), which can be activated by the evaluation device (26), for the emission of defibrillation pulses and in that the electrical pulses generated by the stimulation pulse generator (5) are supplied to the means (22) to determine the impedance measuring signal (Z) from the electrical pulses.

## Revendications

1. Procédé de détection de fibrillations cardiaques ventriculaires par l'exploitation d'un signal (Z) de mesure d'impédance, qui dépend du volume de sang dans le coeur (9), caractérisé par le fait qu'on utilise une diminution du niveau du signal de mesure d'impédance (Z) au-dessous d'un seuil prédéterminé (S) pour la détection de la fibrillation cardiaque ventriculaire.

2. Procédé suivant la revendication 1, caractérisé par le fait qu'on détermine une valeur moyenne dans le temps du niveau du signal de mesure d'impédance (Z) et qu'on règle le seuil (S) à une valeur inférieure, d'une valeur prédéterminée, à la valeur moyenne.

3. Procédé suivant la revendication 1, caractérisé par le fait qu'on utilise la vitesse, à laquelle diminue le niveau du signal de mesure d'impédance (Z), comme critère supplémentaire pour la détection de la fibrillation cardiaque ventriculaire.

4. Procédé suivant la revendication 1 ou 2, caractérisé par le fait qu'on détecte et on évalue la fréquence cardiaque comme critère supplémentaire pour la détection de la fibrillation cardiaque ventriculaire.

5. Dispositif de détection et de traitement de fibrillations cardiaques ventriculaires, comportant un dispositif de mesure d'impédance (5,22) servant à détecter un signal de mesure d'impédance (Z) fonction du volume du sang dans le coeur (9), un dispositif d'exploitation (26) destiné à exploiter le signal de mesure d'impédance (Z) et un dispositif (1), qui produit des impulsions, qui peut être activé par le dispositif d'exploitation (26) pour produire des impulsions électriques et auquel est raccordé, côté sortie, un dispositif d'électrodes (36,37) servant à transmettre les impulsions électriques au coeur (9), caractérisé par le fait que le dispositif d'exploitation (26) comporte un dispositif de détection (27) servant à détecter une diminution du niveau du signal de mesure d'impédance (Z) au-dessous d'un seuil prédéterminé (S).

6. Dispositif suivant la revendication 5, caractérisé par le fait que le dispositif d'exploitation (26) comporte un dispositif de formation de la valeur moyenne servant à former en continu une valeur moyenne dans le temps du niveau du signal de mesure d'impédance (Z) et à régler le seuil (S) à une valeur inférieure, d'une valeur prédéterminée, à la valeur moyenne.

7. Dispositif suivant la revendication 5 ou 6, caractérisé par le fait que le dispositif d'exploitation (26) comporte un autre dispositif de détection (28) destiné à détecter la vitesse, à laquelle diminue le niveau du signal de mesure d'impédance (Z), et des moyens (31) sont prévus pour la combinaison logique des signaux de sortie du dispositif de détection (27) et de l'autre dispositif de détection (28) de telle sorte que la présence simultanée des deux signaux de sortie indique la fibrillation cardiaque ventriculaire.

8. Dispositif suivant l'une des revendications 5 à 7, caractérisé par le fait qu'il est prévu un détecteur de battements cardiaques (14), en aval duquel est branché un autre dispositif d'exploitation (18) servant à déterminer la fréquence cardique, et les signaux de sortie des deux dispositifs d'exploitation (18,26) sont combinés entre eux logiquement de telle sorte que la présence simultanée des deux signaux de sortie indique la fibrillation cardiaque ventriculaire.

9. Dispositif suivant l'une des revendications 5 à 8, caractérisé par le fait que le dispositif de mesure d'impédance (5,22) comporte des moyens (22) pour déterminer le signal de mesure d'impédance (Z) à partir d'impulsions électriques qui sont fournies au coeur (8) par l'unité (5) produisant les impulsions, par l'intermédiaire du dispositif d'électrodes (10,4).

10. Dispositif suivant la revendication 9, caractérisé par le fait que les impulsions électriques, produites pour la détermination du signal de mesure d'impédance (Z), ont une amplitude inférieure au seuil d'excitation du coeur (9).

11. Dispositif suivant la revendication 9 ou 10, caractérisé par le fait que l'unité produisant les impulsions comporte un générateur d'impulsions de stimulation (5) destiné à fournir des impulsions de stimulation cardiaque et un générateur d'impulsions de défibrillation (35) pouvant être activé par le dispositif d'exploitation (26) et destiné à fournir des impulsions de défibrillation, et les impulsions électriques, qui sont produites par le générateur d'impulsions de stimulation (5), sont envoyées aux moyens (22) destinés à déterminer le signal de mesure d'impédance (Z) à partir des impulsions électriques.
